# FASCICULE DE BREVET EUROPEEN

(11) **EP 3 016 701 B1**
(45) Date de publication et mention de la délivrance du brevet: **13.03.2019**
(21) Numéro de dépôt: 14758596.2
(22) Date de dépôt: 04.07.2014
(51) Int. Cl.: A61M 5/20, A61M 5/28

(54) **AUTOINJECTEUR**
AUTOMATISCHER INJEKTOR
AUTO-INJECTOR

(30) Priorité: 05.07.2013 FR 1356616
(43) Date de publication de la demande: 11.05.2016
(73) Titulaire: Aptar France SAS, 27110 Le Neubourg (FR)
(72) Inventeur: FABIEN, David, F-22700 Saint Quay Perros (FR)
(74) Mandataire: CAPRI
(86) Numéro de dépôt international: PCT/FR2014/051737
(87) Numéro de publication internationale: WO 2015/001280

(56) Documents cités:
- WO-A2-2009/010591
- US-A1- 2001 005 781

## Description

La présente invention concerne un autoinjecteur.

Les autoinjecteurs sont bien connus dans l'état de la technique. Ces dispositifs ont principalement pour but de réaliser une injection automatique du contenu d'une seringue à l'intérieur du corps d'un patient. Divers systèmes existent pour rendre automatique la pénétration de l'aiguille dans le corps du patient ainsi que l'injection du produit fluide contenu dans la seringue. Les autoinjecteurs sont des dispositifs relativement complexes qui doivent répondre à un certain nombre d'exigences de contraintes pour être fiables. La robustesse du dispositif, sa maniabilité, et sa facilité d'utilisation pour l'utilisateur sont également des éléments importants. Par ailleurs, la plupart de ces autoinjecteurs étant à usage unique, le coût de fabrication et d'assemblage est également un facteur dont il faut tenir compte.

Il existe de nombreux autoinjecteurs sur le marché, qui présentent toutefois tous un certain nombre d'inconvénients.

Ainsi, pour les autoinjecteurs qui utilisent le même ressort pour d'abord effectuer le piquage puis l'injection, le ressort doit être suffisamment puissant pour garantir la totalité de la phase d'injection. Ceci est d'autant plus vrai qu'au début de la phase d'injection, il faut généralement une force relativement importante pour décoller le piston de la seringue. De ce fait, le ressort exprime sa puissance maximale lors du piquage, ce qui peut rendre cette phase de piquage douloureuse. De plus, avec un tel ressort très puissant lors du piquage, il existe un risque important de casse de la collerette de la seringue, en particulier quand il s'agit de seringue en verre.

Les documents WO 2009/010591 et US 2001/005781 décrivent des dispositifs de l'état de la technique.

La présente invention a pour but de fournir un autoinjecteur qui ne reproduit pas les inconvénients susmentionnés, et qui permet de répondre aux différentes exigences et contraintes importantes pour une utilisation sûre et fiable de l'autoinjecteur.

La présente invention a aussi pour but de fournir un autoinjecteur qui soit fiable et sûr d'utilisation, qui permette de garantir la distribution de la totalité du produit fluide à l'endroit souhaité, et qui soit simple et peu coûteux à fabriquer et à assembler.

La présente invention a donc pour objet un autoinjecteur comportant un corps, un réservoir contenant du produit fluide et comportant un piston et une aiguille, tel qu'une seringue pré-remplie, ledit autoinjecteur comportant une tige de piston adaptée à coopérer avec le piston dudit réservoir, ladite tige de piston étant déplaçable entre une première position de repos, une seconde position de piquage, dans laquelle ladite tige de piston a déplacé ledit réservoir par rapport audit corps pour réaliser le piquage, et une troisième position d'injection dans laquelle ladite tige de piston a déplacé le piston du réservoir pour injecter le produit fluide à travers l'aiguille, un ressort d'actionnement étant prévu pour solliciter ladite tige de piston d'abord vers sa position de piquage puis vers sa position d'injection, l'autoinjecteur comprenant un système de réglage de force exerçant une force sur ladite tige de piston, ladite force étant opposée à la force exercée par ledit ressort d'actionnement sur ladite tige de piston en fin de piquage pour diminuer la force exercée sur ledit réservoir par ladite tige de piston en fin de piquage, et ladite force s'ajoutant à la force exercée par ledit ressort d'actionnement sur ladite tige de piston en début d'injection pour amplifier la force exercée sur ledit piston par ladite tige de piston en début d'injection.

Avantageusement, ledit système de réglage de force comprend deux organes pivotants coopérant avec ladite tige de piston, lesdits organes pivotants étant reliés entre eux par deux éléments élastiques.

Avantageusement, lesdits organes pivotants tournent autour d'axes de rotation parallèles et fixes par rapport audit corps.

Avantageusement, lesdits éléments élastiques sont fixés auxdits organes pivotants au niveau d'axes mobiles parallèles, tels que des tiges ayant deux bords latéraux, formés sur lesdits organes pivotants.

Avantageusement, lorsque la tige de piston se déplace de sa position de repos vers sa position de piquage, lesdits axes mobiles sont disposés en avant desdits axes fixes dans le sens de déplacement de ladite tige de piston, ladite tige de piston, en fin de déplacement vers sa position de piquage, faisant tourner lesdits organes pivotants de telle manière à charger lesdits éléments élastiques, créant ainsi une force de freinage en fin de piquage.

Avantageusement, lorsque la tige de piston se déplace de sa position de piquage vers sa position d'injection, lesdits axes mobiles sont disposés en arrière desdits axes fixes dans le sens de déplacement de ladite tige de piston, lesdits éléments élastiques chargés, en début de déplacement de la tige de piston vers sa position d'injection, faisant tourner lesdits organes pivotants de telle manière à détendre lesdits éléments élastiques, créant ainsi une force d'amplification en début d'injection.

Avantageusement, chaque organe pivotant comprend plusieurs projections adaptées à coopérer avec plusieurs projections radiales de la tige de piston.

Avantageusement, ledit autoinjecteur est déclenché par un bouton axial, un ou plusieurs bouton(s) latéral(ux), ou un manchon coulissant.

Avantageusement, lors du piquage, la tige de piston coopère avec le piston du réservoir pour déplacer ledit réservoir par rapport au corps.

En variante, lors du piquage, la tige de piston comporte un organe élastique, tel qu'un joint torique disposé dans une gorge de la tige de piston, qui coopère avec une collerette du réservoir pour déplacer ledit réservoir par rapport au corps.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
La figure 1 est une vue schématique en perspective éclatée des composants d'un autoinjecteur, selon un premier mode de réalisation avantageux,
Les figures 2 à 11 illustrent de manière schématique en section transversale les séquences successives de la présente invention, selon le mode de réalisation de la figure 1,
Les figures 12 et 13 sont des vues schématiques simplifiées du fonctionnement de la présente invention, respectivement en phase d'amortissement de la fin du piquage et d'amplification du début d'injection,
Les figures 14 à 16 sont des vues schématiques en section transversale de trois variantes d'actionnement de la présente invention,
La figure 17 est une vue schématique en perspective éclatée des composants d'un autoinjecteur, selon un second mode de réalisation avantageux,
La figure 18 est une vue de détail en section transversale du dispositif de la figure 17,
La figure 19 est une vue de détail de côté d'une partie du dispositif de la figure 17,
La figure 20 est une vue similaire à celle de la figure 19, vue de dessous,
Les figures 21 à 25 illustrent de manière schématique, vues en section transversale, les séquences successives de la présente invention, selon le mode de réalisation de la figure 17, et
Les figures 26 à 30 illustrent de manière schématique, vues de côté, les séquences successives des figures 21 à 25.

L'autoinjecteur va être décrit ci-après en référence à deux modes de réalisation avantageux de celui-ci. Il est toutefois à noter que les autoinjecteurs, qui sont des appareils complexes, comprennent plusieurs modules pour réaliser plusieurs fonctions. Ces divers modules peuvent être utilisés séparément et indépendamment les uns des autres, sans être nécessairement combinés aux autres modules, et pourraient notamment être utilisés dans des autoinjecteurs de forme différente de celle représentée sur les dessins. De plus, il est à noter que les dessins sont des vues schématiques qui ne représentent pas forcément la forme exacte des composants d'un autoinjecteur, et ne sont pas forcément à l'échelle, notamment dans des buts de clarté. Par ailleurs, les dessins ne représentent pas forcément tous les éléments constitutifs d'un autoinjecteur, mais seulement les éléments nécessaires au fonctionnement de la présente invention. Ainsi, divers éléments et modules supplémentaires et/ou complémentaires pourraient être associés à l'autoinjecteur représenté sur les figures.

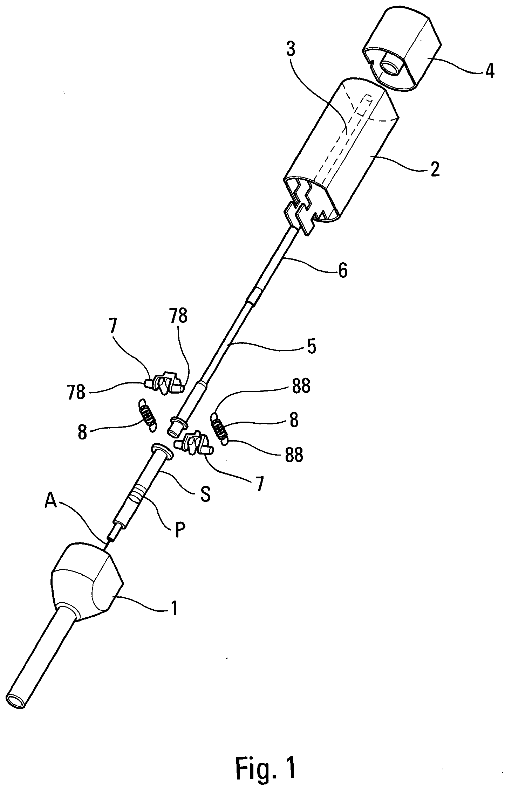

En se référant à la figure 1, les différents composants de l'autoinjecteur, selon un premier mode de réalisation avantageux, sont représentés de manière éclatée.

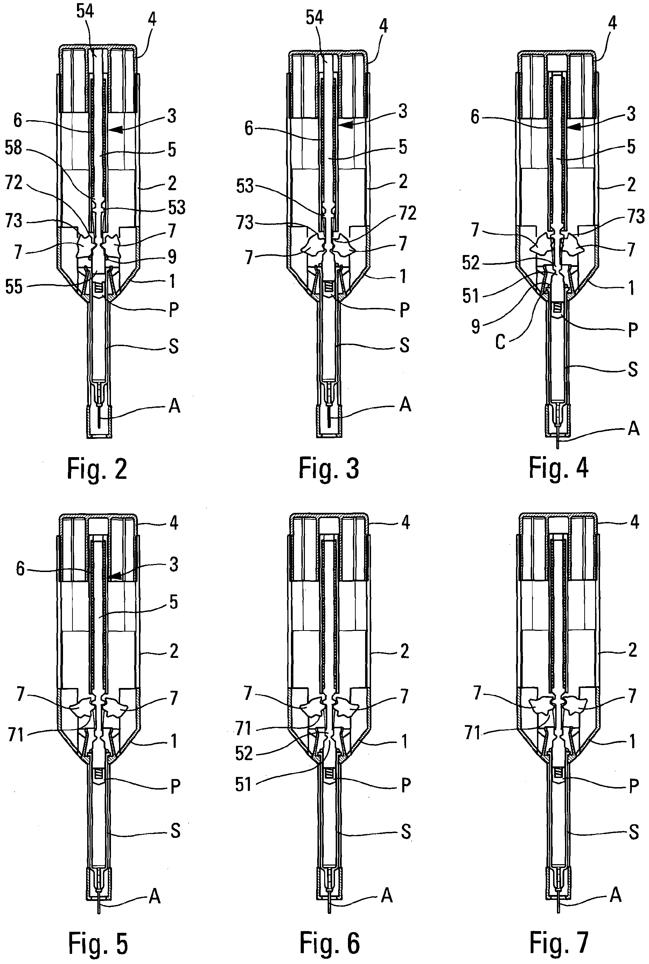

Dans l'ordre des références numériques, l'autoinjecteur comporte un corps inférieur 1, un corps supérieur 2 contenant un manchon 3, un bouton axial d'actionnement 4, une tige de piston 5, un ressort d'actionnement 6, et deux organes pivotants 7, interconnectés au moyen de deux éléments élastiques 8, ici sous forme de ressorts. Ces éléments élastiques 8 sont visibles sur les figures 1, 12 et 13, mais pas sur les figures 2 à 11 et 14 à 16 pour des raisons de clarté.

Il est à noter que les corps inférieur et supérieur pourraient être remplacés par un corps unique.

Un réservoir S peut être inséré dans ledit autoinjecteur, notamment dans son corps inférieur 1. Ce réservoir S contient du produit fluide, et comporte un piston P et une aiguille A. Le piston P est adapté à se déplacer dans ledit réservoir S pour injecter le produit fluide à travers ladite aiguille A.

La présente description sera faite en référence à une seringue S, qui peut être de tout type. Plus généralement, il est entendu que le terme « seringue » dans la présente description englobe tout type de réservoir associé à une aiguille. De préférence, le réservoir S est une seringue pré-remplie.

Le corps inférieur 1 comporte à son extrémité avant (dans le sens de déplacement de la seringue S) une ouverture permettant le passage de l'aiguille A lors de la phase de piquage.

Ce corps inférieur 1 peut comporter des pattes élastiques 15, ou tout autre moyen de retenue, adaptées à retenir la seringue S dans sa position de repos, dans laquelle l'aiguille A ne se projette pas hors dudit corps inférieur. Lors de l'actionnement, ces pattes élastiques 15 libèrent la seringue S qui peut alors se déplacer axialement par rapport au corps inférieur 1 pour permettre à l'aiguille de sortir de celui-ci et de réaliser le piquage.

Le corps supérieur 2 se fixe au corps inférieur, et il peut comporter un manchon central 3 adapté à loger la tige de piston 5 et le ressort 6.

Le bouton axial d'actionnement 4 peut être monté coulissant axialement par rapport au corps supérieur 2, et en contact avec la tige de piston 5. Ainsi, en appuyant sur le bouton axial 4 pour l'enfoncer axialement dans le corps supérieur 2, on déplace axialement la tige de piston 5, ce qui permet le déclenchement du dispositif comme cela sera décrit ci-après.

La tige de piston 5 comporte une extrémité arrière 54 et une extrémité avant 55, dans le sens de déplacement de la tige de piston 5 dans le corps supérieur 2.

Dans cet exemple, l'extrémité arrière 54 coopère avec le bouton axial d'actionnement 4.

L'extrémité avant 55 a pour but de contacter le piston P de la seringue S pour déplacer ledit piston P et ainsi injecter le produit contenu dans la seringue S à travers l'aiguille A.

La tige de piston 5 comporte aussi plusieurs projections radiales 52, 53. Une première projection radiale 52, proximal de l'extrémité avant 55, qui définit une surface avant 51 formant épaulement. Et une seconde projection radiale 53, décalée axialement de ladite première projection radiale 52 en direction de l'extrémité arrière 54, et qui définit une surface avant et une surface arrière. Bien sûr, il ne s'agit là que d'exemples de réalisation, et un homme du métier est capable de réaliser ces épaulements et projections radiales d'une manière différente de celle représentée sur les dessins. En particulier, lesdites projections radiales 52, 53 ne sont pas nécessairement dans la partie avant de la tige de piston 5, comme représenté sur les dessins, mais elles pourraient être réalisées sur une autre partie de la tige de piston 5, par exemple à l'arrière de celle-ci, notamment en arrière de l'appui arrière du ressort d'actionnement 6, dans le sens de déplacement de la tige de piston pendant l'actionnement.

Le ressort d'actionnement 6 s'appuie d'une part dans le manchon 3 du corps supérieur 2 et d'autre part sur la tige de piston, par exemple sur un quatrième épaulement 58 décalé axialement dudit troisième épaulement 53 en direction de l'extrémité arrière 54.

Les organes pivotants 7 sont avantageusement assemblés de manière pivotante sur le corps supérieur 2, et ils sont avantageusement identiques. Ils sont disposés de part et d'autre de la tige de piston 5. Ils ne sont pas déplaçables axialement par rapport audit corps inférieur 1, mais seulement en pivotement autour de leurs axes de rotation 79 qui sont parallèles. En variante, ils pourraient être assemblés différemment, notamment sur le corps inférieur 1.

Chaque organe pivotant 7 comporte plusieurs projections 71, 72, 73. Une première projection 71 adaptée à coopérer avec la surface avant 51 de la première projection radiale 52 de la tige de piston 5. Une seconde projection 72 adaptée à coopérer la surface avant de la seconde projection radiale 53. Et une troisième projection 73 adaptée à coopérer avec la surface arrière de la seconde projection radiale 53. Bien entendu, d'autres mises en oeuvre sont envisageables.

Les éléments élastiques 8 relient les deux organes pivotants 7 entre eux. Le principe consiste à combiner deux axes de rotation fixes, à savoir les axes de rotation 79 des deux organes pivotants 7, avec deux axes mobiles, à savoir les points d'accroche 78 des organes pivotants 7 sur lesquels se fixent les éléments élastiques 8.

Ainsi, comme visible sur la figure 1, le premier élément élastique 8 se fixe d'une part sur un premier point d'accroche du premier organe pivotant et d'autre part sur un premier point d'accroche du second organe pivotant, et il en de même et de manière symétrique pour l'autre élément élastique. De préférence, chaque organe pivotant 7 comporte une tige 78 ayant deux bords latéraux saillants. Le premier élément élastique relie alors les premiers bords saillants entre eux et le second élément élastique relie les seconds bords saillants entre eux. Avantageusement, lorsque les éléments élastiques 8 sont des ressorts, ils sont identiques et comportent des oeillets 88 adaptés à se fixer sur lesdits premiers et seconds bords saillants 78 des organes pivotants 7. En variante, les éléments élastiques pourraient être différents, par exemple sous la forme de joints toriques ou d'autres éléments en matériau élastiquement déformable. L'utilisation d'anneaux en matériau élastique, tels que de joints toriques, en remplacement des ressorts illustrés sur les dessins permettrait notamment de réduire l'encombrement radial du système.

La présente invention a principalement pour objet un système de réglage de force, adapté à exercer une force F1, F2 sur ladite tige de piston 5. La force F1 est opposée à la force exercée par le ressort d'actionnement 6 sur la tige de piston 5 en fin de piquage pour diminuer la force exercée sur ledit réservoir S par ladite tige de piston 5 en fin de piquage. La force F2 s'ajoute au contraire à la force exercée par le ressort d'actionnement 6 sur la tige de piston 5 en début d'injection pour amplifier la force exercée sur ledit piston P par ladite tige de piston 5 en début d'injection.

Ainsi, lorsque les axes mobiles 78 sont décalés axialement par rapport aux axes de rotation fixes 79, ils exercent une force par l'intermédiaire des éléments élastiques 8. Lorsque lesdits axes mobiles 78 sont disposés en avant desdits axes fixes 79 dans le sens de déplacement de la tige de piston 5, cette force va à l'encontre de la rotation impartie auxdits organes pivotants 7 par ladite tige de piston 5. Dans ce cas, la rotation des organes pivotants 7 est donc freinée par lesdits éléments élastiques 8. Au contraire, lorsque lesdits axes mobiles 78 sont disposés en arrière desdits axes fixes 79 dans le sens de déplacement de la tige de piston 5, cette force va dans le sens de la rotation impartie auxdits organes pivotants 7 par ladite tige de piston 5. Dans ce cas, la rotation des organes pivotants 7 est donc amplifiée par lesdits éléments élastiques 8. Lorsque les axes fixes 79 et mobiles 78 sont alignés, on se trouve à un point neutre, dans lequel lesdits éléments élastiques 8 n'influencent pas la rotation des organes pivotants 7. C'est dans cette position que le système bascule d'un état de « freinage » ou « d'amortissement » à un état « d'amplification ».

Les figures 2 à 11 illustrent les séquences d'actionnement de l'autoinjecteur de la figure 1.

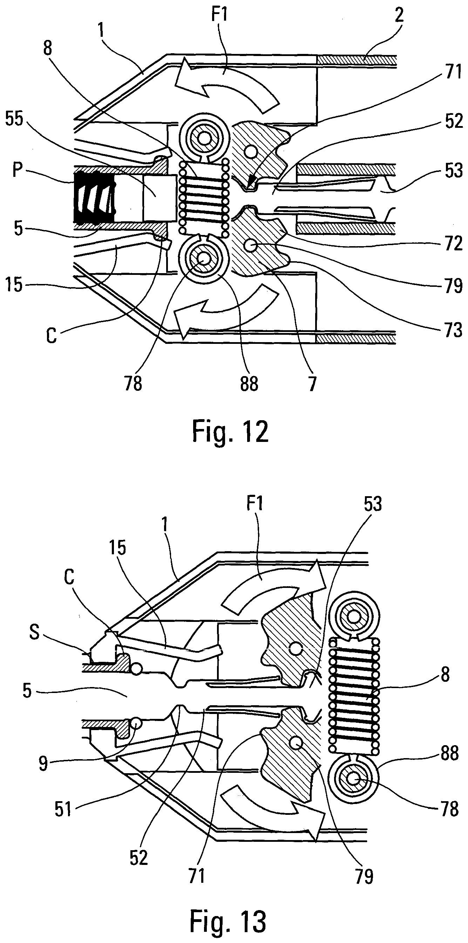

Sur la figure 2, l'autoinjecteur est en position de repos avant actionnement. Dans cette position de repos, l'aiguille A de la seringue S est disposée à l'intérieur du corps inférieur 1. Lesdits axes mobiles 78 sont disposés en avant desdits axes fixes 79 dans le sens de déplacement de la tige de piston 5, comme représenté schématiquement sur la figure 12. Le ressort d'actionnement 6 sollicite la tige de piston 5 en direction du piston P de la seringue S, mais la tige de piston 5 est retenue dans la position de repos par la première projection 71 des organes pivotants 7 qui coopère avec le premier épaulement 51 de la tige de piston 5. Sous la pression du ressort d'actionnement 6, ledit premier épaulement 51 de la tige de piston 5 pousse la première projection 71 des organes pivotants 7 en rotation, mais cette rotation est contrée par les éléments élastiques 8, dont la force est choisie de manière appropriée pour assurer une position de repos stable. Ces éléments élastiques 8 contrebalancent donc la force du ressort d'actionnement 6, et sans intervention extérieure, le dispositif est bloqué en position de repos.

Lorsque l'utilisateur veut utiliser l'autoinjecteur, il prend le dispositif, par exemple au niveau du corps supérieur 2 et il appuie sur le bouton axial d'actionnement 4. Ce faisant, il déplace légèrement la tige de piston 5 axialement, ce qui provoque la rotation des organes pivotants 7.

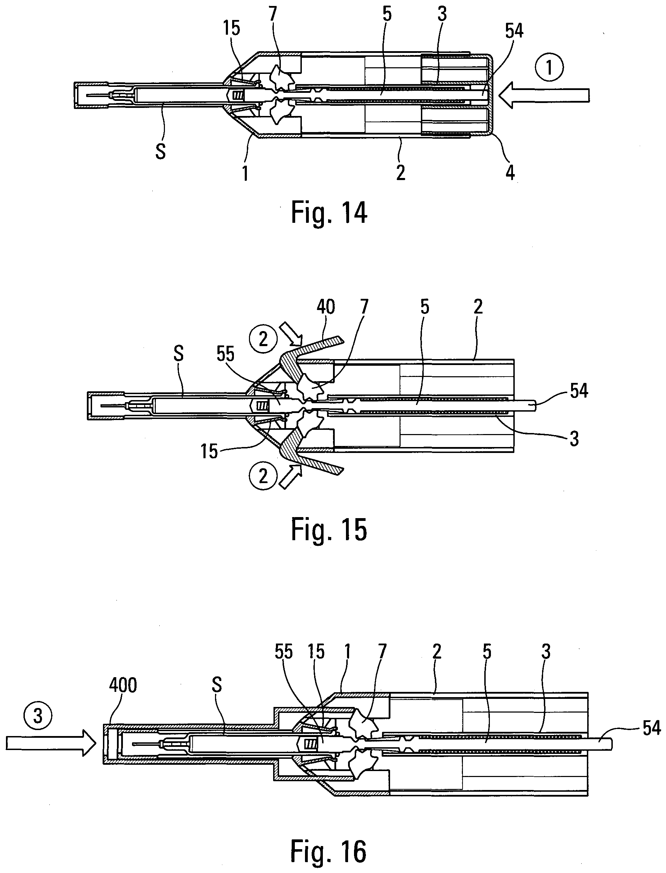

Des variantes de déclenchement sont représentées sur les figures 14 à 16. La figure 14 illustre le déclenchement par un bouton axial 4 comme décrit ci-dessus, et implique l'application d'une force de déclenchement axiale dans le sens de la flèche 1. La figure 15 illustre une variante dans laquelle la force de déclenchement est appliquée latéralement dans le sens de la ou des flèche(s) 2, par un ou deux boutons latéraux 40. La figure 16 illustre une autre variante dans laquelle la force de déclenchement est appliquée axialement dans le sens de la flèche 3, par un manchon coulissant 400 disposé autour du corps inférieur 1 en contact avec le site d'injection.

La rotation des organes pivotants 7 générée par la force d'actionnement provoque le désengrènement entre la première projection 71 et le premier épaulement 51. Ceci libère donc la tige de piston 5, qui est alors déplacée axialement sous l'effet du ressort d'actionnement 6. Ceci provoque le déplacement de la seringue S dans le corps inférieur 1 et donc le piquage, comme illustré sur les figures 3 et 4.

Le déplacement de la seringue S peut être réalisé par contact entre l'extrémité avant 55 de la tige de piston et le piston P de la seringue S. En variante, comme illustré notamment sur les figures 3, 4 et 13, un organe élastique 9, tel qu'un joint torique, peut être monté sur la tige de piston 5, par exemple dans une gorge appropriée, cet organe élastique 9 coopérant avec la collerette C de la seringue S lors de la phase de piquage, puis se désolidarisant de ladite tige de piston 5 lors de la phase d'injection. Cette mise en oeuvre évite un contact entre la tige de piston et le piston pendant le piquage.

Pendant cette phase de piquage, les premières projections 71 et/ou les secondes projections 72 des organes pivotants 7 vont glisser le long de la tige de piston 5 entre lesdites première et seconde projections radiales 52, 53. Dans l'exemple représenté, les premières projections 71 glissent le long de la tige de piston 5 entre lesdites première et seconde projections radiales 52, 53, alors que les projections 72 ne rencontrent pas d'obstacles car elles sont situées dans des évidements appropriés de la tige de piston 5. Eventuellement, les organes pivotants 7 peuvent continuent de pivoter légèrement lors de ce coulissement, par exemple via des rampes inclinées formées sur la tige de piston 5 entre la première projection radiale 52 et la seconde projection radiale 53. Ceci permet de fournir un léger amortissement ou freinage lors du piquage.

Lorsque la seconde projection 72 des organes pivotants atteint la surface avant de la seconde projection radiale 53 de la tige de piston 5, la phase de piquage n'est pas totalement terminée. Ceci est visible sur la figure 4, qui montre que la collerette C de la seringue est encore à une petite distance de la partie du corps inférieur qui va définir sa position de piquage. A ce moment-là, la surface avant de la seconde projection radiale 53 va faire tourner davantage les organes pivotant 7 en poussant sur leurs secondes projections 72. Ceci va encore davantage tendre ou charger les éléments élastiques 8, qui vont donc opposer une force croissante à la rotation des organes pivotants 7. Cette force de « freinage » ou « d'amortissement » est représentée par la flèche F1 sur la figure 12. Ceci génère un amortissement en fin de piquage, en diminuant la force exercée par la tige de piston 5 sur la seringue S, ce qui améliore grandement le confort de l'utilisateur et évite d'endommager la collerette C de la seringue S. Ceci est visible sur les figures 5 à 7. Bien entendu, les forces du ressort d'actionnement 6 et des éléments élastiques 8 sont choisies de telle sorte que le piquage n'est qu'amortit sans être stoppé.

Au fur et à mesure que les organes pivotants 7 tournent lors de la phase de piquage, les éléments élastiques 8 se tendent de plus en plus. Simultanément, les axes mobiles 78 des organes pivotants 7 se rapprochent progressivement des axes fixes 79. Le dispositif est ajusté avantageusement pour générer un couple maximal au moment de (ou juste avant) la fin de la phase de piquage. Le point neutre dans lequel les axes mobiles et fixes sont alignés peut donc être atteint au moment de (ou juste avant) la fin de la phase de piquage.

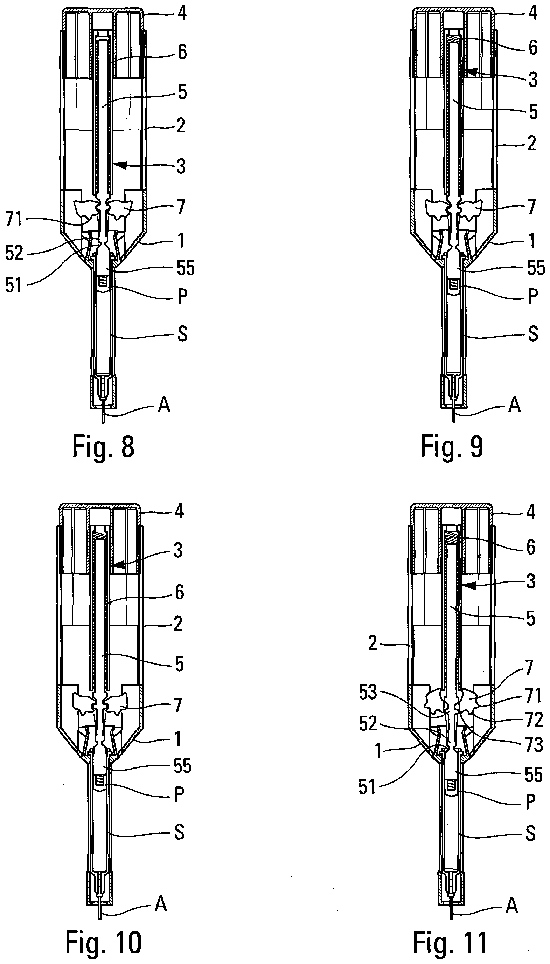

Lorsque les organes pivotants 7 et les éléments élastiques 8 sont dans la position neutre, représentée sur la figure 8, la tige de piston est toujours sollicitée axialement par le ressort d'actionnement 6. Cette position neutre n'est donc pas stable, et le système bascule automatiquement de l'état de freinage du piquage vers l'état d'amplification du début de l'injection. Eventuellement, la troisième projection 73 peut être disposée par rapport à la seconde projection 72 de telle sorte que les organes pivotants 7 effectuent juste après le passage de la position neutre une légère rotation sous l'effet des éléments élastiques 8 tendus. Ceci peut permettre de générer un son audible lorsque ladite troisième projection 73 va heurter la seconde projection radiale 53 de la tige de piston, pour indiquer à l'utilisateur le début de la phase d'injection.

Lorsque l'aiguille A atteint sa position de piquage avec une insertion complète de l'aiguille, la phase d'injection est déclenchée, ce qui est représenté sur les figures 9 à 11. L'extrémité avant 55 de la tige de piston va alors pousser sur le piston P sous l'effet de la force exercée par le ressort d'actionnement 6. Pendant toute la phase d'injection, la tige de piston 5 coulisse à l'intérieur de la seringue S en poussant le piston P de celle-ci sous l'effet du ressort 6. Le produit est ainsi distribué à travers l'aiguille A.

Au début de la phase d'injection, la troisième projection 73 de chaque organe pivotant 7 va donc venir en contact de la surface arrière de la seconde projection radiale 53. Comme en fin de phase de piquage, le couple exercé par le système est maximal juste après la position neutre, et les éléments élastiques 8 tendus sollicitent donc fortement les organes pivotants 7 en rotation. Ceci a pour effet d'amplifier la force du ressort d'actionnement 6 en début de phase d'injection. Cette force d'amplification est illustrée par la flèche F2 sur la figure 13. Cette amplification augmente la force exercée par la tige de piston 5 sur le piston P, et permet ainsi de garantir le décollement du piston P de sa position de repos, sans avoir à augmenter la force du ressort d'actionnement 6. En effet, la résistance maximale lors de la phase d'injection est créée au moment du décollement du piston P. Une fois que l'injection a débutée, les frottements du piston P dans la seringue S, la viscosité du produit à injecter et la résistance du passage étroit de l'aiguille A sont inférieurs et ne nécessitent donc plus la même force du ressort d'actionnement 6.

Comme visible sur la figure 11, les organes pivotants 7 se désengrènent de la tige de piston après une course d'injection relativement petite de la tige de piston 5, typiquement de quelques millimètres, par exemple environ 4 mm dans l'exemple représenté. A partir de ce désengrènement, le système devient inactif, et l'injection du produit se poursuit normalement. Eventuellement, on peut prévoir que les organes pivotants 7 poursuivent une rotation après désengrènement, par exemple pour générer un son audible par l'utilisateur. Il est aussi envisageable d'adapter le système de réglage de force pour qu'il puisse amplifier l'effort exercé sur le piston P pendant une partie de course d'injection plus grande, par exemple environ 20 mm, voire pendant la totalité de la course d'injection, notamment avec des réservoirs dont la dimension axiale serait réduite.

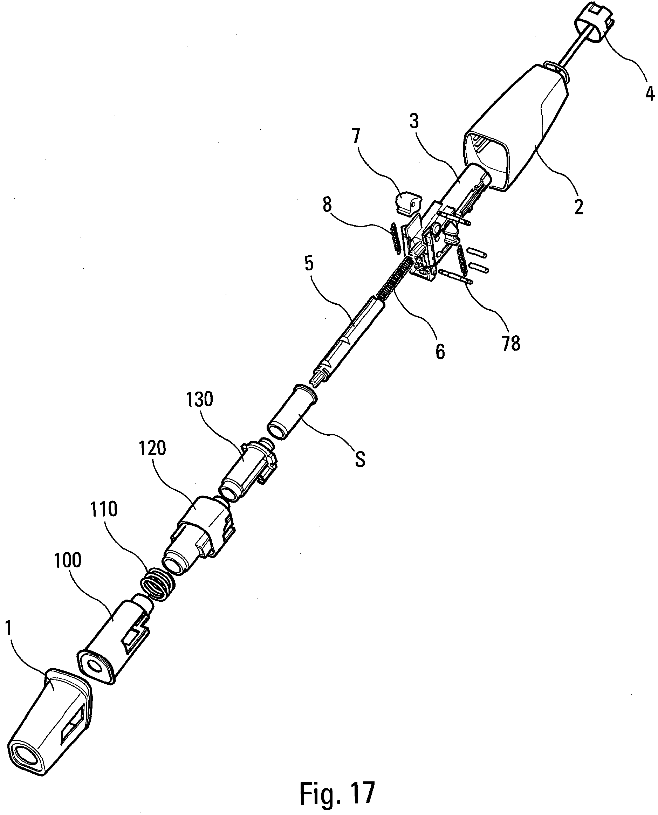

En se référant à la figure 17, les différents composants de l'autoinjecteur, selon un second mode de réalisation avantageux, sont représentés de manière éclatée.

Ce second mode de réalisation est similaire au premier mode de réalisation décrit précédemment, et seules les différences par rapport à ce premier mode de réalisation seront explicitées plus en détails ci-après, le fonctionnement global étant similaire sinon identique entre les deux modes de réalisation. Les éléments similaires ou identiques ont donc les mêmes références numériques dans les deux modes de réalisation.

Comme visible sur la figure 17, ce second mode de réalisation diffère principalement du premier mode de réalisation par la présence, dans le corps inférieur, d'un manchon actionneur 100, adapté à coulisser par rapport audit corps inférieur 1 entre une position initiale de repos, avant actionnement, dans laquelle il se projette axialement hors dudit corps inférieur 1, une position actionnée, dans laquelle il est déplacé axialement vers l'intérieur dudit corps inférieur 1, et une position finale de sécurité, dans laquelle il est à nouveau projeté hors dudit corps inférieur, pour recouvrir l'aiguille de la seringue S après injection. Il est à noter que la position finale de sécurité peut être identique à la position initiale de repos, ou en variante, ces deux positions peuvent être différentes, avec par exemple le manchon actionneur s'étendant axialement plus loin hors dudit corps inférieur 1 dans ladite position finale de sécurité par rapport à ladite position initiale de repos. Ce manchon actionneur 100 est avantageusement sollicité axialement vers l'extérieur dudit corps inférieur 1 par un ressort 110, de sorte qu'avant actionnement, le manchon actionneur 100 est sollicité vers sa position initiale de repos, et après actionnement, le manchon actionneur 100 est sollicité vers sa position finale de sécurité.

Le corps inférieur 1 peut en outre contenir un corps interne 120 recevant un élément de support de réservoir 130 dans lequel est insérée la seringue S.

Dans ce second mode de réalisation, Les organes pivotants 7 sont avantageusement assemblés de manière pivotante sur le manchon 3. En variante, ils pourraient être assemblés différemment, notamment sur le corps inférieur 1 ou sur le corps supérieur 2.

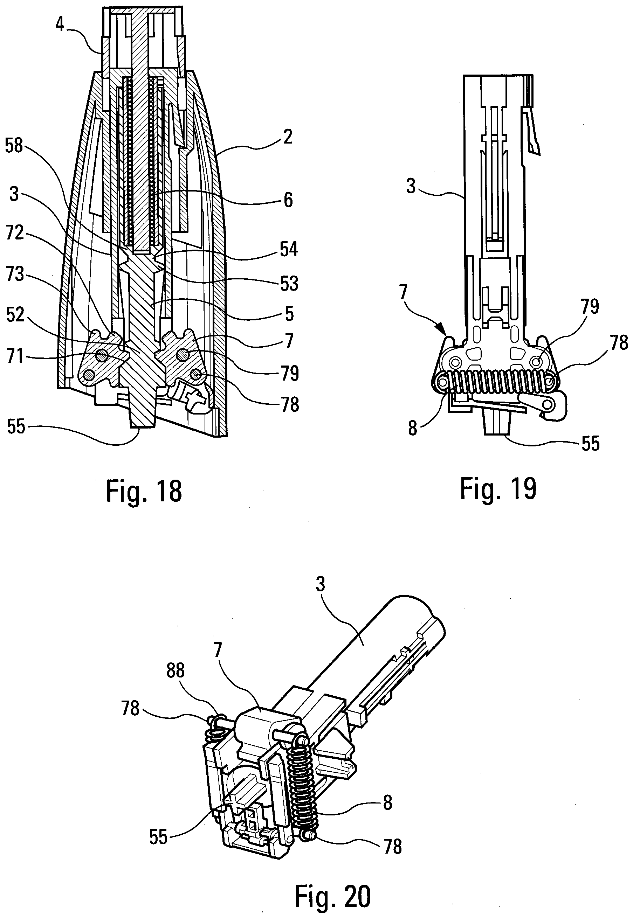

Les figures 18 à 20 illustrent l'agencement particulier de ce second mode de réalisation, et notamment celui des organes pivotants 7 et des éléments élastiques 8, qui sont également des ressorts dans cet exemple.

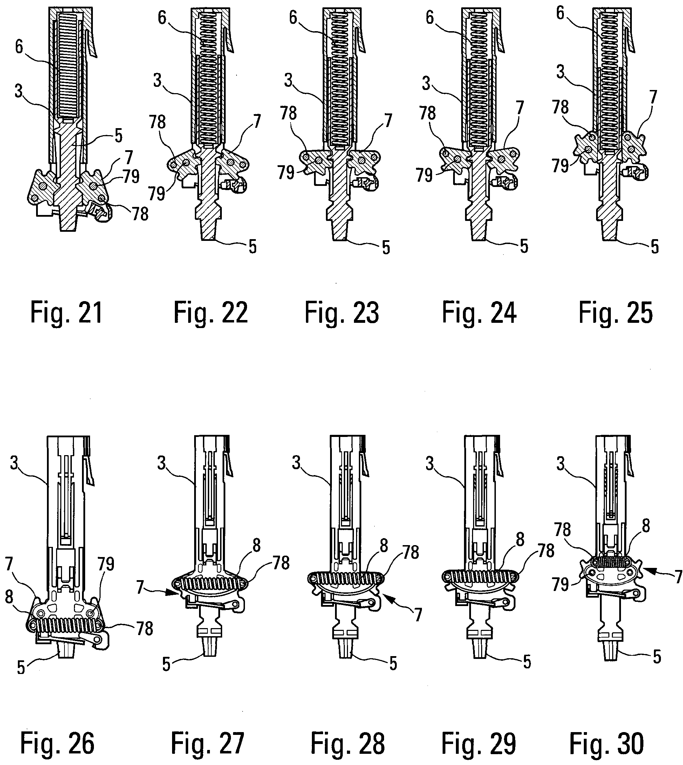

La séquence de fonctionnement est représentée d'une part sur les figures 21 à 25, vue en section transversale avec les ressorts non visibles, et sur les figures 26 à 30, qui montrent l'état du ressort 8 dans les différentes positions des figures 21 à 25.

Ainsi, les figures 21 et 26 montrent la position de repos ou de stockage, avant actionnement. Dans les figures 22 et 27, la phase de piquage est en cours, et le pivotement des organes pivotants 7 provoque l'augmentation de la tension des ressorts 8, ce qui engendre l'effet de freinage décrit précédemment. Dans les figures 23 et 28, les organes pivotants sont au point neutre, avec les ressorts 8 tendus au maximum. Dans les figures 24 et 29, la phase d'injection débute, avec l'effet d'amplification décrit précédemment généré par les ressorts 8 qui se détendent. Enfin les figures 25 et 30 illustrent les organes pivotants 7 en position désengrenée avec les ressorts 8 détendus.

Typiquement, des forces F1 d'amortissement et F2 d'amplification de l'ordre de 30N sont possibles. Bien entendu, d'autres valeurs d'amortissement et de freinage peuvent être obtenues en choisissant de manière appropriée les éléments élastiques 8 et en dimensionnant de manière appropriée les organes pivotants 7.

Il est à noter que la force de freinage F1 en fin de piquage n'est pas obligatoire, et que la présente invention peut ne prévoir que la force d'amplification F2 en début d'injection. Dans ce cas, les organes pivotants 7 et les éléments élastiques 8 sont agencés de telle sorte qu'ils n'exercent que ledit effet d'amplification au début de la phase d'injection. L'application d'une force de freinage F1 en fin de piquage est donc optionnelle.

La présente invention s'applique à des dispositifs utilisés notamment pour les traitements des maladies auto-immunes, par exemple du type arthrites rhumatoïdes, scléroses multiples, maladie de Crohn, pour les traitements contre le cancer, pour les traitements antiviraux, par exemple du type hépatites, pour les traitements contre le diabète, pour les traitements contre l'anémie ou pour les traitements de crises, par exemple en cas de choc anaphylactique.

Bien que la présente invention ait été décrite en référence à deux modes de réalisation avantageux, il est entendu que diverses modifications sont possibles pour l'homme du métier sans sortir du cadre de la présente invention tel que défini par les revendications annexées.

## Revendications

1. Autoinjecteur comportant un corps (1, 2), un réservoir (S) contenant du produit fluide et comportant un piston (P) et une aiguille (A), tel qu'une seringue pré-remplie, ledit autoinjecteur comportant une tige de piston (5) adaptée à coopérer avec le piston (P) dudit réservoir (S), ladite tige de piston (5) étant déplaçable entre une première position de repos, une seconde position de piquage, dans laquelle ladite tige de piston (5) a déplacé ledit réservoir (S) par rapport audit corps (1, 2) pour réaliser le piquage, et une troisième position d'injection dans laquelle ladite tige de piston (5) a déplacé le piston (P) du réservoir (S) pour injecter le produit fluide à travers l'aiguille (A), un ressort d'actionnement (6) étant prévu pour solliciter ladite tige de piston (5) d'abord vers sa position de piquage puis vers sa position d'injection, **caractérisé en ce que** l'autoinjecteur comprend un système de réglage de force (7, 8) exerçant une force (F1, F2) sur ladite tige de piston (5), ladite force (F1) étant opposée à la force exercée par ledit ressort d'actionnement (6) sur ladite tige de piston (5) en fin de piquage pour diminuer la force exercée sur ledit réservoir (S) par ladite tige de piston (5) en fin de piquage, et ladite force (F2) s'ajoutant à la force exercée par ledit ressort d'actionnement (6) sur ladite tige de piston (5) en début d'injection pour amplifier la force exercée sur ledit piston (P) par ladite tige de piston (5) en début d'injection, ledit système de réglage de force (7, 8) comprenant deux organes pivotants (7) coopérant avec ladite tige de piston (5), lesdits organes pivotants (7) étant reliés entre eux par deux éléments élastiques (8).

2. Autoinjecteur selon la revendication 1, dans lequel lesdits organes pivotants (7) tournent autour d'axes de rotation (79) parallèles et fixes par rapport audit corps (1, 2).

3. Autoinjecteur selon la revendication 1 ou 2, dans lequel lesdits éléments élastiques (8) sont fixés auxdits organes pivotants (7) au niveau d'axes mobiles parallèles (78), tels que des tiges ayant deux bords latéraux, formés sur lesdits organes pivotants (7).

4. Autoinjecteur selon les revendications 2 et 3, dans lequel, lorsque la tige de piston (5) se déplace de sa position de repos vers sa position de piquage, lesdits axes mobiles (78) sont disposés en avant desdits axes fixes (79) dans le sens de déplacement de ladite tige de piston (5), ladite tige de piston (5), en fin de déplacement vers sa position de piquage, faisant tourner lesdits organes pivotants (7) de telle manière à charger lesdits éléments élastiques (8), créant ainsi une force de freinage (F1) en fin de piquage.

5. Autoinjecteur selon la revendication 4, dans lequel, lorsque la tige de piston (5) se déplace de sa position de piquage vers sa position d'injection, lesdits axes mobiles (78) sont disposés en arrière desdits axes fixes (79) dans le sens de déplacement de ladite tige de piston (5), lesdits éléments élastiques chargés (8), en début de déplacement de la tige de piston (5) vers sa position d'injection, faisant tourner lesdits organes pivotants (7) de telle manière à détendre lesdits éléments élastiques (8), créant ainsi une force d'amplification (F2) en début d'injection.

6. Autoinjecteur selon l'une quelconque des revendications précédentes, dans lequel chaque organe pivotant (7) comprend plusieurs projections (71, 72, 73) adaptées à coopérer avec plusieurs projections radiales (52, 53) de la tige de piston (5).

7. Autoinjecteur selon l'une quelconque des revendications précédentes, dans lequel ledit autoinjecteur est déclenché par un bouton axial (4), un ou plusieurs boutons latéraux (40), ou un manchon coulissant (400).

8. Autoinjecteur selon l'une quelconque des revendications précédentes, dans lequel lors du piquage, la tige de piston (5) coopère avec le piston (P) du réservoir (S) pour déplacer ledit réservoir (S) par rapport au corps (1, 2).

9. Autoinjecteur selon l'une quelconque des revendications 1 à 7, dans lequel lors du piquage, la tige de piston (5) comporte un organe élastique (9), tel qu'un joint torique disposé dans une gorge de la tige de piston, qui coopère avec une collerette (C) du réservoir (S) pour déplacer ledit réservoir (S) par rapport au corps (1, 2).

## Patentansprüche

1. Autoinjektor, umfassend einen Körper (1, 2), einen Behälter (S), der ein fluides Produkt enthält und einen Kolben (P) und eine Nadel (A), wie eine vorgefüllte Spritze, umfasst, wobei der Autoinjektor eine Kolbenstange (5) umfasst, die dafür geeignet ist, mit dem Kolben (P) des Behälters (S) zusammenzuwirken, wobei die Kolbenstange (5) zwischen einer ersten Ruheposition, einer zweiten Stechposition, in der die Kolbenstange (5) den Behälter (S) in Bezug auf den Körper (1, 2) verschoben hat, um das Stechen zu bewirken, und einer dritten Injektionsposition, in der die Kolbenstange (5) den Kolben (P) des Behälters (S) verschoben hat, um das fluide Produkt durch die Nadel (A) zu injizieren, verschiebbar ist, wobei eine Betätigungsfeder (6) vorgesehen ist, um die Kolbenstange (5) zunächst in ihre Stechposition, dann in ihre Injektionsposition zu belasten, **dadurch gekennzeichnet, dass** der Autoinjektor ein Kraftregelungssystem (7, 8) umfasst, das eine Kraft (F1, F2) auf die Kolbenstange (5) ausübt, wobei die Kraft (F1) der Kraft entgegengesetzt ist, die am Ende des Stechens von der Betätigungsfeder (6) auf die Kolbenstange (5) ausgeübt wird, um die Kraft zu verringern, die am Ende des Stechens von der Kolbenstange (5) auf den Behälter (S) ausgeübt wird, und die Kraft (F2) zu der Kraft hinzukommt, die zu Beginn der Injektion von der Betätigungsfeder (6) auf die Kolbenstange (5) ausgeübt wird, um die Kraft zu verstärken, die zu Beginn der Injektion von der Kolbenstange (5) auf den Kolben (P) ausgeübt wird, wobei das Kraftregelungssystem (7, 8) zwei schwenkbare Organe (7) umfasst, die mit der Kolbenstange (5) zusammenwirken, wobei die schwenkbaren Organe (7) durch zwei elastische Elemente (8) miteinander verbunden sind.

2. Autoinjektor nach Anspruch 1, wobei sich die schwenkbaren Organe (7) um Drehachsen (79) drehen, die parallel und in Bezug auf den Körper (1, 2) fixiert sind.

3. Autoinjektor nach Anspruch 1 oder 2, wobei die elastischen Elemente (8) an mobilen parallelen Achsen (78), wie Stangen mit zwei seitlichen Rändern, die auf den schwenkbaren Organen (7) ausgebildet sind, an den schwenkbaren Organen (7) befestigt sind.

4. Autoinjektor nach den Ansprüchen 2 und 3, wobei, wenn sich die Kolbenstange (5) aus ihrer Ruheposition in ihre Stechposition verschiebt, die mobilen Achsen (78) in Verschiebungsrichtung der Kolbenstange (5) vor den fixierten Achsen (79) angeordnet sind, wobei die Kolbenstange (5) am Ende der Verschiebung in ihre Stechposition die schwenkbaren Organe (7) derart dreht, dass die elastischen Elemente (8) belastet werden, wodurch eine Bremskraft (F1) am Ende des Stechens erzeugt wird.

5. Autoinjektor nach Anspruch 4, wobei, wenn sich die Kolbenstange (5) von ihrer Stechposition in ihre Injektionsposition verschiebt, die beweglichen Achsen (78) in Verschiebungsrichtung der Kolbenstange (5) hinter den fixierten Achsen (79) angeordnet sind, wobei die elastischen belasteten Elemente (8) zu Beginn der Verschiebung der Kolbenstange (5) in ihre Injektionsposition die schwenkbaren Organe (7) derart drehen, dass die elastischen Elemente (8) entspannt werden, wodurch eine Verstärkungskraft (F2) zu Beginn der Injektion erzeugt wird.

6. Autoinjektor nach einem der vorhergehenden Ansprüche, wobei jedes schwenkbare Organ (7) mehrere Vorsprünge (71, 72, 73) umfasst, die dazu geeignet sind, mit mehreren radialen Vorsprüngen (52, 53) der Kolbenstange (5) zusammenzuwirken.

7. Autoinjektor nach einem der vorhergehenden Ansprüche, wobei der Autoinjektor durch einen axialen Knopf (4), einen oder mehrere seitliche Knöpfe (40) oder eine Gleitmuffe (400) ausgelöst wird.

8. Autoinjektor nach einem der vorhergehenden Ansprüche, wobei die Kolbenstange (5) während des Stechens mit dem Kolben (P) des Behälters (S) zusammenwirkt, um den Behälter (S) in Bezug auf den Körper (1, 2) zu verschieben.

9. Autoinjektor nach einem der Ansprüche 1 bis 7, wobei die Kolbenstange (5) während des Stechens ein elastisches Organ (9) umfasst, wie einen O-Ring, der in einer Rille der Kolbenstange angeordnet ist, das mit einem Kragen (C) des Behälters (S) zusammenwirkt, um den Behälter (S) in Bezug auf den Körper (1, 2) zu verschieben.

## Claims

1. An autoinjector comprising a body (1, 2), and a reservoir (S) containing fluid and including a piston (P) and a needle (A), such as a prefilled syringe, said autoinjector further comprising a piston rod (5) that is suitable for co-operating with the piston (P) of said reservoir (S), said piston rod (5) being movable between a first rest position, a second pricking position, in which said piston rod (5) has moved said reservoir (S) relative to said body (1, 2) so as to perform pricking, and a third injection position in which said piston rod (5) has moved the piston (P) of the reservoir (S) so as to inject the fluid through the needle (A), an actuator spring (6) being provided so as to urge said piston rod (5) firstly towards its pricking position and then towards its injection position, the autoinjector being **characterized in that** it further comprises a force-adjustment system (7, 8) that exerts a force (F1, F2) on said piston rod (5), said force (F1) opposing the force exerted by said actuator spring (6) on said piston rod (5) at the end of pricking so as to decrease the force exerted on said reservoir (S) by said piston rod (5) at the end of pricking, and said force(F2) adding to the force exerted by said actuator spring (6) on said piston rod (5) at the beginning of injection, so as to amplify the force exerted on said piston (P) by said piston rod (5) at the beginning of injection, said force-adjustment system (7, 8) comprising two pivot members (7) that co-operate with said piston rod (5), said pivot members (7) being connected together by two resilient elements (8).

2. An autoinjector according to claim 1, wherein said pivot members (7) pivot about pivot pins (79) that are parallel and stationary relative to said body (1, 2).

3. An autoinjector according to claim 1 or claim 2, wherein said resilient elements (8) are fastened to said pivot members (7) via parallel movable pins (78), such as rods having two side edges, that are formed on said pivot members (7).

4. An autoinjector according to claims 2 and 3, wherein, when the piston rod (5) moves from its rest position towards its pricking position, said movable pins (78) are arranged in front of said stationary pins (79) in the travel direction of said piston rod (5), said piston rod (5), at the end of travel towards its pricking position, causing said pivot members (7) to pivot in such a manner as to load said resilient elements (8), thereby creating a braking force (F1) at the end of pricking.

5. An autoinjector according to claim 4, wherein, when the piston rod (5) moves from its pricking position towards its injection position, said movable pins (78) are arranged behind said stationary pins (79) in the travel direction of said piston rod (5), said loaded resilient elements (8), at the beginning of travel of the piston rod (5) towards its injection position, causing said pivot members (7) to pivot in such a manner as to relax said resilient elements (8), thereby creating an amplification force (F2) at the beginning of injection.

6. An autoinjector according to any preceding claim, wherein each pivot member (7) includes a plurality of projections (71, 72, 73) that are suitable for co-operating with a plurality of radial projections (52, 53) of the piston rod (5).

7. An autoinjector according to any preceding claim, wherein said autoinjector is triggered by an axial button (4), one or more side buttons (40), or a slidable sleeve (400).

8. An autoinjector according to any preceding claim, wherein, during pricking, the piston rod (5) co-operates with the piston (P) of the reservoir (S) so as to move said reservoir (S) relative to the body (1, 2).

9. An autoinjector according to any one of claims 1 to 7, wherein, during pricking, the piston rod (5) includes a resilient member (9), such as an O-ring arranged in a groove of the piston rod, that co-operates with a collar (C) of the reservoir (S) so as to move said reservoir (S) relative to the body (1, 2).
